# EUROPEAN PATENT APPLICATION

(11) **EP 1 517 145 A1**
(43) Date of publication of application: **23.03.2005**
(21) Application number: 04254883.4
(22) Date of filing: 13.08.2004
(51) Int. Cl.: G01N 33/53, B01J 19/00

(54) **Selectable length linear microarrays**

(30) Priority: 13.08.2003 US 640801
(71) Applicant: Agilent Technologies, Inc., Palo Alto, CA 94306 (US)
(72) Inventor: Schembri, Carol T., San Mateo California 94403 (US)
(74) Representative: Tollett, Ian

(57) **Abstract**

Devices (10) are disclosed comprising a substrate in the form of an elongated web having on a surface thereof a linear array of chemical compounds and markings on the elongated web indicating segments of the linear array comprising groups of one or more chemical compounds of the linear array. In certain embodiments the devices (10) comprise a severable housing (12), a linear array (16) of features (18) within the housing, and markings (20) on the housing indicating segments (24) of the linear array comprising groups of one or more features of the linear array. Usually, the housing is an enclosed microchannel (14). The device may be employed for conducting an assay for one or more analytes suspected of being in a sample.

## Description

The present invention relates to linear arrays and, particularly, to linear microarrays of selectable length. More particularly, the invention relates to linear microarrays of biopolymers from which a user can select a desired length for a particular application. In addition, the invention relates to a method for conducting an assay using such a microarray and a method for preparing a device comprising such a microarray.

Determining the nucleotide sequences and expression levels of nucleic acids (DNA and RNA) is critical to understanding the function and control of genes and their relationship, for example, to disease discovery and disease management. Analysis of genetic information plays a crucial role in biological experimentation. This has become especially true with regard to studies directed at understanding the fundamental genetic and environmental factors associated with disease and the effects of potential therapeutic agents on the cell. Such a determination permits the early detection of infectious organisms such as bacteria, viruses, etc.; genetic diseases such as sickle cell anemia; and various cancers. New methods of diagnosis of diseases, such as AIDS, cancer, sickle cell anemia, cystic fibrosis, diabetes, muscular dystrophy, and the like, rely on the detection of mutations present in certain nucleotide sequences. This paradigm shift has lead to an increasing need within the life science industries for more sensitive, more accurate and higher-throughput technologies for performing analysis on genetic material obtained from a variety of biological sources.

Unique or misexpressed nucleotide sequences in a polynucleotide can be detected by hybridization with a nucleotide multimer, or oligonucleotide, probe. Hybridization reactions between surface-bound probes and target molecules in solution may be used to detect the presence of particular biopolymers. Hybridization is based on complementary base pairing. When complementary single stranded nucleic acids are incubated together, the complementary base sequences pair to form double stranded hybrid molecules. These techniques rely upon the inherent ability of nucleic acids to form duplexes via hydrogen bonding according to Watson-Crick base-pairing rules. The ability of single stranded deoxyribonucleic acid (ssDNA) or ribonucleic acid (RNA) to form a hydrogen-bonded structure with a complementary nucleic acid sequence has been employed as an analytical tool in molecular biology research. An oligonucleotide probe employed in the detection is selected with a nucleotide sequence complementary, usually exactly complementary, to the nucleotide sequence in the target nucleic acid. Following hybridization of the probe with the target nucleic acid, any oligonucleotide probe/nucleic acid hybrids that have formed are typically separated from unhybridized probe. The amount of oligonucleotide probe in either of the two separated media is then tested to provide a qualitative or quantitative measurement of the amount of target nucleic acid originally present.

Such reactions form the basis for many of the methods and devices used in the field of genomics to probe nucleic acid sequences for novel genes, gene fragments, gene variants and mutations. The ability to clone and synthesize nucleotide sequences has led to the development of a number of techniques for disease diagnosis and genetic analysis. Genetic analysis, including correlation of genotypes and phenotypes, contributes to the information necessary for elucidating metabolic pathways, for understanding biological functions, and for revealing changes in genes that confer disease. Many of these techniques generally involve hybridization between a target nucleotide sequence and a complementary probe, offering a convenient and reliable means for the isolation, identification, and analysis of nucleotides. The surface-bound probes may be oligonucleotides, peptides, polypeptides, proteins, antibodies or other molecules capable of reacting with target molecules in solution.

Direct detection of labeled target nucleic acid hybridized to surface-bound polynucleotide probes is particularly advantageous if the surface contains a mosaic of different probes that are individually localized to discrete, known areas of the surface. Such ordered arrays of probes are commonly referred to as 'biochip" arrays. Biochip arrays containing a large number of oligonucleotide probes have been developed as tools for high throughput analyses of genotype and gene expression. Oligonucleotides synthesized on a solid support recognize uniquely complementary nucleic acids by hybridization, and arrays can be designed to define specific target sequences, analyze gene expression patterns or identify specific allelic variations.

In one approach, cell matter is lysed, to release its DNA as fragments, which are then separated out by electrophoresis or other means, and then tagged with a fluorescent or other label. The resulting DNA mix is exposed to an array of oligonucleotide probes, whereupon selective attachment to matching probe sites takes place. The array is then washed and imaged so as to reveal for analysis and interpretation the sites where attachment occurred.

One typical method involves hybridization with probe nucleotide sequences immobilized in an array on a substrate having a surface area of typically less than a few square centimeters. The substrate may be glass, fused silica, silicon, plastic or other material; preferably, it is a glass slide, which has been treated to facilitate attachment of the probes. The mobile phase, containing reactants that react with the attached probes, is placed in contact with the substrate, covered with another slide, and placed in an environmentally controlled chamber such as an incubator. Normally, the reactant targets in the mobile phase diffuse through the liquid to the interface where the complementary probes are immobilized, and a reaction, such as a hybridization reaction, then occurs. Preferably, the mobile phase targets are labeled with a detectable tag, such as a fluorescent tag, or chemiluminescent tag, or radioactive label, so that the reaction can be detected. The location of the signal in the array provides the target identification. The hybridization reaction typically takes place over a time period of seconds up to many hours.

Biochip arrays have become an increasingly important tool in the biotechnology industry and related fields. These binding agent arrays, in which a plurality of binding agents are synthesized on or deposited onto a substrate in the form of an array or pattern, find use in a variety of applications, including gene expression analysis, drug screening, nucleic acid sequencing, mutation analysis, and the like. Substrate-bound biopolymer arrays, particularly oligonucleotide, DNA and RNA arrays, may be used in screening studies for determination of binding affinity and in diagnostic applications, e.g., to detect the presence of a nucleic acid containing a specific, known oligonucleotide sequence.

The pattern of binding by target molecules to biopolymer probe spots on the biochip forms a pattern on the surface of the biochip and provides desired information about the sample. Hybridization patterns on biochip arrays are typically read by optical means, although other methods may also be used. For example, laser light in the Agilent Technologies Inc. GeneArray Scanner excites fluorescent molecules incorporated into the nucleic acid probes on a biochip, generating a signal only in those spots on the biochip that have a target molecule bound to a probe molecule, thus generating an optical hybridization pattern. This pattern may be digitally scanned for computer analysis. Such patterns can be used to generate data for biological assays such as the identification of drug targets, single-nucleotide polymorphism mapping, monitoring samples from patients to track their response to treatment, and assess the efficacy of new treatments.

A linear array is a one-dimensional array of features bound in a non-diffusive manner to a surface usually located on the inside of an enclosed microchannel. The order of the features identifies each feature, which allows selective identification of target molecules. One such linear array is disclosed in U.S. Patent No. 5,804,384 (Muller, *et al*.), the relevant disclosure of which is incorporated herein by reference. The devices of Muller, *et al*., consist of a tube containing a linear array of specific binding elements that each have capture probes specific for a target analyte. In one approach the device includes a linear array of binding elements layered in a one-dimensional stack in the lumen of a tube, such as a capillary tube. The binding elements in this format can consist of any standard column-packing material. For example, glass microbeads, fritted glass, sintered glass, silicon, agarose beads, glass wool, or a gel, such as a polyacrylamide gel, can be used. Thus, binding elements can be made up of multiple, discrete subunits, such as beads, that are each linked to the same binding factor.

Usually, a linear array has a fixed length determined by the number of features of the linear array. The linear array typically includes many more features than a user might find necessary to use. The cost and other factors associated with the preparation of linear arrays that are customized for a particular user are generally much greater than that associated with preparing general linear arrays that include a fixed number of features. The user employs the fixed length arrays for the determination of one or more analytes, which are many times far less than the total number of analytes that could be determined by the linear array. The user focuses on results pertaining to the desired analytes and ignores those results that fall outside of area of interest.

It is desirable to have a linear array of fixed length that a user could selectively sever into test devices for particular analytes of interest to the user. In this way, generation of needless data may be avoided. Furthermore, determinations involving test devices that the user has severed into a desired length uses much less sample suspected of containing the analytes than the amount of sample that would be employed on an unsevered linear array. Accordingly, a desirable conservation of sample may be achieved.

One embodiment of the present invention is a device comprising a severable housing, a linear array of features within the housing, and markings on the housing indicating segments of the linear array comprising groups of one or more features of the linear array. Usually, the housing is an enclosed microchannel. The device may be employed for conducting an assay for one or more analytes suspected of being in a sample.

Another embodiment of the present invention is a method for conducting an assay for one or more biopolymers suspected of being in a sample. The method employs a device that is severed to obtain a segment comprising features for specifically identifying the one or more biopolymers. The device comprises a severable housing, a linear array of the features within the housing, and markings on the housing indicating segments comprising the segment. The features of the severed segment are contacted with the sample and the biopolymers that have become bound to the features are determined.

Another embodiment of the present invention is a method of preparing a device for conducting an assay for one or more analytes. A linear microarray of features is formed within a severable housing. At least a portion of the features comprises a segment for detecting the one or more analytes. The housing is marked to indicate segments, at least one of which segments comprises the portion of features.

Another embodiment of the present invention is a method of preparing a device for conducting an assay for one or more analytes. A linear array of features is formed on a flexible substrate wherein at least a portion of the features is for detecting the one or more analytes. The flexible substrate is sealed to form a channel comprising the linear array.

The following figures are included to better illustrate the embodiments of the devices and techniques of the present invention. The figures are not to scale and some parts of the figures may be exaggerated for the purpose of illustrating certain aspects or embodiments of the present invention. Preferred embodiments of the present invention will now be described, by way of example only, with reference to the Figures:
Fig. 1 is a perspective view taken from the top of a portion of an embodiment of a device in accordance with the present invention.
Fig. 2 is a cross-sectional view of the device of Fig. 1 taken alone lines 2-2.

As mentioned above, embodiments of the present invention are directed to a device comprising a linear array of features on an elongated web, usually as part of a housing such as, for example, an enclosed microchannel. The device is constructed to be severable into segments as desired by the user. The device comprises markings to identify the segments, which may be viewed as multiple arrays, for easy and accurate severing of the overall device by the user.

As mentioned above, a linear array is a one-dimensional array of features bound in a non-diffusive manner to a surface. By the term "non-diffusive" is meant that the molecules that make up the individual features are bound to the surface in such a manner that they will not detach under the conditions of preparing and using the linear array. Non-diffusive binding may be covalent or may be non-covalent or macromolecular association where the linking is of sufficient strength to withstand the aforementioned conditions. Non-diffusive binding of the features may be achieved in a number of approaches known in the art. Some of those approaches are discussed briefly hereinbelow by way of illustration and not limitation.

The features generally are molecules that are involved in the detection of target molecules or analytes in a sample of interest. Each molecule of a feature may be specific for a corresponding analyte or for a compound indicative of the presence of the analyte. For example, the analyte may be part of a complex such as, for example, an antigen-antibody complex, polynucleotide-protein complex, polynucleotide-polynucleotide complex and the like, and the feature is capable of binding to a component of the complex. Usually, the molecule comprising the feature is a specific binding partner for the analyte or for a member of the complex indicative of the presence of the analyte. The members of a pair of molecules (e.g., a detector probe or a capture probe and a target analyte, or the members of a specific binding pair (e.g., antibody-antigen, nucleic acid, and protein-vitamin binding pairs)) are said to "specifically bind" to each other if they bind to each other with greater affinity than to other, non-specific molecules. For example, an antibody raised against an antigen to which it binds more efficiently than to a non-specific antigen can be described as specifically binding to the antigen. Similarly, a nucleic acid probe can be described as specifically binding to a nucleic acid target if it forms a specific duplex with the target by base pairing interactions.

Each feature, or element, within the linear array is defined to be a small, regularly shaped region of the surface of the substrate. The features in the linear array are arranged in a predetermined manner. Each feature of a linear array usually carries a predetermined chemical compound or mixtures thereof and is typically of homogeneous composition. Each feature within the linear array may contain a different molecular species, and the molecular species within a given feature may differ from the molecular species within the remaining features of the molecular array. Some or all of the features may be of different compositions. Each array may be separated by spaces or areas. Interarray areas and interfeature areas are usually present but are not essential. These interarray and interfeature areas do not carry any chemical compound such as polynucleotide (or other biopolymer of a type of which the features are composed). Interarray areas and interfeature areas typically will be present where arrays are formed by the conventional *in situ* process or by deposition of previously obtained moieties, as described herein, by depositing for each feature at least one droplet of reagent such as from a pulse jet but may not be present when, for example, photolithographic array fabrication processes are used. It will be appreciated though that the interarray areas and interfeature areas, when present, could be of various sizes and configurations.

In the linear array the order of the features identifies each feature, which allows selective identification of target molecules. Usually, the linear array has a fixed length determined by the number of features of the linear array. The width of the linear array is usually one feature. However, for purposes of the present invention, the width of the linear array may be greater than one feature where the size of the feature and the width of the housing, e.g., microchannel, permit. Therefore, the width of the linear array may be 1 to about 5 features, 1 to about 4 features, 1 to about 3 features, 1 to 2 features. In such an embodiment where the linear array is more than one feature wide, each feature comprising the width at the position in question may be the same or different and each feature comprising the length of the linear array may be the same or different, usually different, as discussed above. The width of the features, for example, the diameter of a round spot, may be in the range from about 10 µm to about 1.0 cm. In other embodiments each feature may have a width in the range of about 1.0 µm to about 1.0 mm, usually about 5.0 µm to about 500 µm, and more usually about 10 µm to about 200 µm. Non-round features may have width ranges equivalent to that of circular features with the foregoing width (diameter) ranges.

The housing for the linear array is any enclosure in which the linear array may be formed or situated. The housing may be a microchannel. In one approach, the microchannel is part of a microfluidic system. Microfluidic systems have been developed for performing chemical, clinical, and environmental analysis of chemical and biological specimens. The term microfluidic system refers to a system or device having a network of chambers connected by channels, in which the channels have microscale features, that is, features too small to examine with the unaided eye. The channel often has a capillary dimension, i.e., a cross-sectional area that provides for capillary flow through the channel. At least one of the cross-sectional dimensions, e.g., width, height, diameter, is at least about 1 µm, usually at least about 10 µm, and is usually no more than about 500 µm, preferably no more than about 200 µm. Channels of capillary dimension typically have an inside bore diameter (ID) of from about 1 to about 200 microns, more typically from about 25 to about 100 microns. The term "microfluidic" generally means of or pertaining to fluids and being of a magnitude on the order consistent with capillary dimension. The channel(s) may be part of a microfluidic network or a system of interconnected cavity structures and capillary-size channels configured with a plurality of branches through which fluids may be manipulated and processed. However, in its simplest and preferred form, the present devices comprise a microchannel in the form of a tube within a housing.

Such microfluidic systems are often fabricated using photolithography, wet chemical etching, and other techniques similar to those employed in the semiconductor industry. The resulting devices can be used to perform a variety of sophisticated chemical and biological analytical techniques.

The channel is thus a conduit by which a sample may contact a linear array. The channels, and thus the linear array, may be straight, curved, serpentine, labyrinth-like or other convenient configuration comprised of separate tubes or part of a monolithic, often planar, substrate. The cross-sectional shape of the channel is not critical and may be circular, ellipsoid, square, rectangular, triangular and the like. The inside of the channel may be coated with a material for strength, for enhancing or reducing electrokinetic flow, for enhancing detection limits and sensitivity, and so forth. Exemplary of coatings are silylation, polyacrylamide (vinyl bound), methylcellulose, polyether, polyvinylpyrrolidone, and polyethylene glycol, polypropylene, Teflon™ (DuPont), Nafion™ (DuPont), and the like may also be used.

The channel usually comprises an entry port, namely, any site at which a liquid may be introduced into a device having one or more channels. The entry port may be a well or simply the terminus of a channel that opens any place on the device such as at an edge.

Moving materials through microchannels may be accomplished, for example, by use of a fluid pressure difference, by use of various electro-kinetic processes including electrophoresis, electroosmotic flow, and electrokinetic pumping, and so forth. Microfluidic devices generally include one or more channels fabricated on or within the devices, usually within the devices. The devices also can include reservoirs, fluidly connected to the channels, which can be used to introduce materials into the channels to contact a linear array contained in the channel. Microfluidic systems have a number of advantages over conventional chemical or physical laboratory techniques. For example, microfluidic systems are particularly well adapted for analyzing small sample sizes, typically making use of samples on the order of nanoliters and even picoliters. The substrates may be produced at relatively low cost, and the channels can be arranged to perform numerous specific analytical operations, including mixing, dispensing, valving, reactions, detections, electrophoresis, and the like. The analytical capabilities of such microfluidic systems may be enhanced by increasing the number and complexity of network channels, reaction chambers, and the like. However, for the purposes of the present invention involving linear arrays that are severable into segments for analysis of a limited number of analytes, the microfluidic system is often less complex.

As mentioned above, the length of the linear array as manufactured is usually a fixed length determined by the number of features of the linear array. In addition, as mentioned above, because of factors such as, for example, cost and ease of manufacturing, general fixed length linear arrays are most cost effective. The number of features is related to the nature of the features, the nature of the analytes, the complexity of the biological or clinical questions being investigated, the number of quality control features desired, and so forth. A typical linear array may contain more than about ten, more than about one hundred, more than about one thousand, more than about ten thousand, more than about twenty thousand, etc., more than about one hundred thousand, features and so forth.

The housing for the linear array in accordance with the present invention is severable. This means that the housing for the linear array is fabricated from a material or substrate that may be readily cut by the user into sections with a cutting instrument such as, for example, a knife, scissors, blade, and the like or readily broken into sections with the use of minimal force. Accordingly, the housing is distinguished from one that is not readily severable into sections. For purposes of the present invention, a material for the housing is not readily severable into sections if the severing requires saws, laser cutting tools, machinists equipment such as lathe or mill, and the like. It should be understood that the material from which the housing is fabricated may be flexible or rigid where rigid material is treated to render it severable. To this end, the housing for the linear array is considered severable within the meaning of the present invention if the material for the housing is rendered breakable along a line such as a score line or the like.

The material for the housing should provide physical support for the chemical compounds that are deposited on an interior surface of the housing or synthesized on an interior surface of the housing *in situ* from subunits. The materials should be of such a composition that they endure the conditions of a deposition process and/or an *in situ* synthesis and of any subsequent treatment or handling or processing that may be encountered in the use of a particular array.

Typically, the housing material is transparent or comprises a viewing area that is transparent. By "transparent" is meant that the substrate material permits signal from features on an interior surface of the substrate to pass therethrough without substantial attenuation and also permits any interrogating radiation to pass therethrough without substantial attenuation. By "without substantial attenuation" may include, for example, without a loss of more than about 40% or more preferably without a loss of more than about 30%, about 20% or about 10%, of signal. The interrogating radiation and signal may for example be visible, ultraviolet or infrared light. In certain embodiments, such as for example where production of binding pair arrays for use in research and related applications is desired, the materials from which the substrate may be fabricated should ideally exhibit a low level of non-specific binding during hybridization events. Alternatively, the material may be opaque if the covering forming the top of channel comprising the linear array is removed or opened prior to a scanning for optical signal or if non-optical detection methods are employed such as radiation.

The materials may be naturally occurring or synthetic or modified naturally occurring. Suitable flexible materials include, for example, flexible plastics, flexible resins, and laminates or composites of plastics and very thin layers of glass, oxides or metals that are thin enough to be flexible, and so forth. Particular flexible plastics finding use include, for example, polyethylene, polypropylene, polytetrafluoroethylene (PTFE), e.g., TEFLON®, polymethylmethacrylate, polycarbonate, polyethylene terephthalate, polystyrene or styrene copolymers, polyurethanes, polyesters, polycarbonates, polyureas, polyamides, polyethyleneamines, polyarylene sulfides, polysiloxanes, polydimethylsiloxanes, polyimides, polyacetates, poly etheretherketone (PEEK), and the like, either used alone or in conjunction with another material or materials provided that the overall composition is flexible and severable.

Suitable rigid materials may include glass, which term is used to include silica including, for example, glass such as glass available as Bioglass, and suitable rigid plastics and resins, and so forth. Rigid plastics include, for example, polymers such as, e.g., poly (vinyl chloride), polyacrylamide, polyacrylate, polyethylene, polypropylene, poly(4-methylbutene), polystyrene, polymethacrylate, poly(ethylene terephthalate), nylon, poly(vinyl butyrate), etc., either used by themselves or in conjunction with other materials.

The housing comprising the linear array may be prepared in a number of ways. The following discussion is by way of illustration and not limitation. In one approach, the linear array is synthesized or deposited on the surface of a housing substrate and the area comprising at least the linear array is enclosed to form a channel comprising the linear array.

In one embodiment, the linear array may be synthesized or deposited on the surface of a substrate in the dimensions desired. For example, for a microarray the chemical compounds comprising the linear array are synthesized or deposited in an area that corresponds to capillary dimensions. The substrate may be flexible and may be substantially flat along the area of synthesis or deposition or there may be a groove, depression, or the like in the substrate where the linear array is placed. The term "web" as used herein refers to a long continuous piece of substrate material having a length greater than a width. For example, the web length to width ratio may be at least 5/1, 10/1, 50/1, 100/1, 200/1, or 500/1, or even at least 1000/1. In this way the web may be considered to be elongated.

To form a housing comprising the linear array, the area of deposition or synthesis on the substrate or web ultimately may be enclosed to form a channel having the linear array therein. Enclosing the aforementioned area of the substrate or web to form a housing with a channel comprising the linear array may be accomplished in a number of ways. One important consideration in enclosing the housing is to avoid damage to the linear array on the surface of the housing substrate. In one approach, for example, the substrate is a flexible material that is folded or rolled over to enclose the housing to form the channel. After folding, the flexible material is sealed to itself in an area outside the area of the channel. Sealing may be achieved by application of heat, adhesives, ultrasonic welding, solvent bonding, and so forth.

The parameters for sealing are dependent on the nature of the flexible material, the nature of the sealing method, compatibility with the bioprobes on the surface and the subsequent assay, and the like. For heat sealing, where the flexible material is a flexible plastic, the material is heated at a temperature and for a time to achieve adequate sealing of the material. Heating parameters are dependent on the nature of the material, the thickness of the material, the applied pressure, and the like.

Various adhesives may be employed to seal the substrate material. The primary consideration for the adhesive is that it be compatible with the reagents employed in any assay in which the linear arrays are employed. Such adhesives include, for example, epoxies, acrylics, urethanes, and so forth, as long as they are compatible with the assay.

In an alternate approach, a separate material may be placed over the substrate comprising the linear array and sealed to the substrate to enclose the housing to form the channel with the linear array therein. The separate material may be sealed to the flexible substrate as discussed above. The separate material may have the same composition as the substrate or a composition that is different from the substrate. A primary consideration is that the separate material is severable as discussed above.

As mentioned above, a rigid material may require scoring to render it severable by the user. To score a rigid material for the housing, the material may be subjected to a procedure in which minute fissures or cracks are propagated into the body of the material usually to a depth sufficient to obtain a clean break. However, the score line should not be so deep that there is a risk of the material breaking prior to the time desired by the user. The depth of the fissures depends on the type of material and the thickness of the material. Usually, for glass this depth is about 100 to about 500 microns, more usually, about 150 to about 250 microns. Any cutter or cutting means may be employed that can provide the score lines at predetermined positions along the housing for the linear array. For example, the housing may be scored using a conventional diamond or tungsten carbide wheel, which is drawn across the housing in the desired locations to form score lines. Other examples of ways in which the material may be scored include laser scribing, laser ablation, laser perforation, water jet ablation, and the like. A rigid housing for the linear array is usually scored after depositing and/or synthesizing chemical compounds in the form of arrays on an interior surface of the housing although scoring before such deposition or synthesis may be employed in some instances.

The interior surface of the housing to which a plurality of chemical compounds is attached to form the linear array can be hydrophilic or capable of being rendered hydrophilic or it may be hydrophobic. The interior surface is normally treated to create a primed or functionalized surface, that is, a surface that is able to support the attachment of a fully formed chemical compound or the synthetic steps involved in the production of the chemical compound on the surface of the substrate. Functionalization relates to modification of the surface of a substrate to provide a plurality of functional groups on the substrate surface. By the term "functionalized surface" is meant a substrate surface that has been modified so that a plurality of functional groups are present thereon usually at discrete sites on the surface. The manner of treatment is dependent on the nature of the chemical compound to be synthesized or deposited and on the nature of the surface. In one approach a reactive hydrophilic site or reactive hydrophilic group is introduced onto the surface of the substrate. Such hydrophilic moieties can be used as the starting point in a synthetic organic process.

The surface of the housing onto which the chemical compounds are deposited or formed may be modified with one or more different layers of compounds that serve to modify the properties of the surface in a desirable manner. Such modification layers, when present, will generally range in thickness from a monomolecular thickness to about 1 mm, usually from a monomolecular thickness to about 0.1 mm and more usually from a monomolecular thickness to about 0.001 mm. Modification layers of interest include: inorganic and organic layers such as metals, metal oxides, polymers, small organic molecules and the like. Polymeric layers of interest include layers of: peptides, proteins, polynucleic acids or mimetics thereof (for example, peptide nucleic acids and the like); polysaccharides, phospholipids, polyurethanes, polyesters, polycarbonates, polyureas, polyamides, polyethylene amines, polyarylene sulfides, polysiloxanes, polyimides, polyacetates, and the like, where the polymers may be hetero- or homopolymeric, and may or may not have separate functional moieties attached thereto (for example, conjugated). Various further modifications to the particular embodiments described above are, of course, possible. Accordingly, the present invention is not limited to the particular embodiments described in detail above.

As mentioned above, the chemical compounds that are bound to the interior surface of the housing to form the linear array may be synthesized or deposited on the surface. Usually, an initial derivatization of the surface is carried out. Modification of surfaces for use in chemical synthesis has been described. See, for example, U.S. Patent No. 5,266,222 (Willis) and U.S. Patent No. 5,137,765 (Farnsworth).

The arrays may be, and are usually, microarrays created on the interior surface of the housing by *in situ* synthesis of biopolymers such as polynucleotides, polypeptides, polysaccharides, etc., and combinations thereof, or by deposition of molecules such as oligonucleotides, cDNA and so forth. In general, arrays are synthesized on a surface by one of any number of synthetic techniques that are known in the art.

In one embodiment, the surface of the substrate is siliceous, i.e., the surface comprises silicon oxide groups, either present in the natural state or introduced by techniques well known in the art. One technique for introducing siloxyl groups onto the surface involves reactive hydrophilic moieties on the surface. These moieties are typically epoxide groups, carboxyl groups, thiol groups, and/or substituted or unsubstituted amino groups as well as a functionality that may be used to introduce such a group such as, for example, an olefin that may be converted to a hydroxyl group by means well known in the art. One approach is disclosed in U.S. Patent No. 5,474,796 (Brennan), the relevant portions of which are incorporated herein by reference. A siliceous surface may be used to form silyl linkages, i.e., linkages that involve silicon atoms. Usually, the silyl linkage involves a silicon-oxygen bond, a silicon-halogen bond, a silicon-nitrogen bond, or a silicon-carbon bond.

Another method for attachment is described in U.S. Patent No. 6,219,674 (Fulcrand, *et al*.). A surface is employed that comprises a linking group consisting of a first portion comprising a hydrocarbon chain, optionally substituted, and a second portion comprising an alkylene oxide or an alkylene imine wherein the alkylene is optionally substituted. One end of the first portion is attached to the surface and one end of the second portion is attached to the other end of the first portion chain by means of an amine or an oxy functionality. The second portion terminates in an amine or a hydroxy functionality. The surface is reacted with the substance to be immobilized under conditions for attachment of the substance to the surface by means of the linking group.

Another method for attachment is described in U.S. Patent No. 6,258,454 (Lefkowitz, *et al*.). A solid substrate having hydrophilic moieties on its surface is treated with a derivatizing composition containing a mixture of silanes. A first silane provides the desired reduction in surface energy, while the second silane enables functionalization with molecular moieties of interest, such as small molecules, initial monomers to be used in the solid phase synthesis of oligomers, or intact oligomers. Molecular moieties of interest may be attached through cleavable sites.

A procedure for the derivatization of a metal oxide surface uses an aminoalkyl silane derivative, e.g., trialkoxy 3-aminopropylsilane such as aminopropyltriethoxy silane (APS), 4-aminobutyltrimethoxysilane, 4-aminobutyltriethoxysilane, 2-aminoethyltriethoxysilane, and the like. APS reacts readily with the oxide and/or siloxyl groups on metal and silicon surfaces. APS provides primary amine groups that may be used to carry out the present methods. Such a derivatization procedure is described in EP 0 173 356 B1, the relevant portions of which are incorporated herein by reference. Other methods for treating the surface of a substrate to which the chemical compounds become bound will be suggested to those skilled in the art in view of the teaching herein.

The housing comprising the linear array may be provided in any number of convenient forms depending on the nature of the housing, the nature of the linear array, the number of probes, the type of test being conducted, and so forth. In one attractive approach, the arrays are provided in the form of sheet or tubing and wrapped around a spool and so forth. In one attractive approach, the housing is provided in the form of a spool, which is conveniently un-spooled by the user and cut as desired. In other approaches the linear array may be provided as separate arrays in a stiff tube or sheet form or individually packaged and the like.

In a specific embodiment the housing is manufactured from a flexible substrate, which may be the same as or similar to that described in U.S. Patent Application Serial No. 10/037757, entitled "Chemical Arrays" by Schembri, *et al*., filed Oct. 18, 2001, published as U.S. Patent Publication No. 20030108726 and U.S. Patent Application Serial No. 10/032608, entitled "Chemical Arrays", by Lefkowitz, *et al*., filed Oct. 18, 2001, published as U.S. Patent Publication No. 20030077380. The flexible substrate is a plastic, that is, any synthetic organic polymer of high molecular weight (for example at least 1,000 grams/mole, or even at least 10,000 or 100,000 grams/mole. With regard to the present invention, and as discussed above, the flexible substrate must be severable by the user.

In one embodiment in accordance with the above disclosure, the flexible substrate may have a number of different layers. A base layer forms the greatest thickness and may consist of any flexible plastic such as a polyolefin film (such as polypropylene, polyethylene, polymethylpentene) or polyetheretherketone, polyimide, any of the fluorocarbon polymers or other suitable flexible thermoplastic polymer film. The material of the base layer is best selected to provide stable dimensional, mechanical, and chemical properties as well as severability. For example, for polynucleotide arrays the flexible substrate is subject to elevated temperatures (for example, 60°C) for long times (for example, 12 hours) in aqueous environments. Polyester or aramid films exposed to such conditions may tend to swell or degrade. When the type of linear arrays and the conditions to which the layer will be exposed are selected, the base layer can be selected for dimensional, mechanical and chemical stability under such conditions by reference to many known polymer film characteristic sources. The base layer will typically have a thickness of more than about 1 µm (or more than about 5 µm) and less than about 500 µm (or even less than about 100, about 50, about 25, or about 15 µm).

The flexible substrate may also include an optional reflective layer and a transparent layer. The reflective layer may be aluminum, silver, gold, platinum, chrome or other suitable metal film deposited by vacuum deposition, plasma enhanced chemical vapor deposition or other means onto the base layer or an optional intermediate bonding layer. Alternatively, the reflective layer may be constructed using multiple dielectric layers designed as a dielectric Bragg reflector or the like. Typically, such a reflector is constructed by repeating ¼ wave thick layers of two optically clear dielectric materials that have differing indices of refraction. Design considerations for such a reflector include the excitation and emission wavelengths and the angle of incidence for the excitation beam and detector. The composition and/or thickness of the reflective layer is such that the overall flexible substrate is severable as discussed above. Although many of the materials from which the reflective layer may be made are rigid at a certain thickness, the thickness of the reflective layer for purposes of the present invention is such that the overall flexible substrate is severable. Accordingly, the thickness of the reflective layer is usually less than about 50 nm, or even less than about 20, about 10, about 5 or about 1nm but in any case, for example, more than about 0.1 or about 0.5 nm. The reflective layer may particularly be chosen to have a thickness such that it is opaque to the wavelength of the light used for illuminating the features during array reading.

A bonding layer, if used, may be any suitable material that is flexible at the thickness used and bonds to the base layer and/or the reflective layer. The bonding layer may have a thickness of less than about 50 nm, or even less than about 20, about 10, about 5 or about 1nm and usually more than about 0.1 or about 0.5 nm).

A glass layer (which term is used to include silica) may be deposited onto the reflective layer by sputtering, plasma enhanced chemical vapor deposition or similar techniques such as described in. A glass layer may optionally be used without a reflective layer. Several manufacturers have commercial capabilities for providing films coated with metal and glass layers, for example, Sheldahl Corporation, Northfield, MN (see the world wide web site at www.sheldahl.com), and General Atomic, San Diego, CA (world wide web site address of ga.com). The glass layer has a thickness such that the overall flexible substrate is severable. Accordingly, the thickness of the glass layer may be, for example, greater than about 1, about 10 or about 100 nm, and less than about 1000, about 700, or about 400 nm but typically has a thickness about ¼ wavelength of the light used to illuminate array features during reading, or an odd multiple of that amount. For example, about 40 to about 200 nm, or about 60 to about 120nm (or even about 80 to about 100nm), or an odd integer multiple of any of the foregoing thickness ranges (for example, about 300nm may be used) provided the layer is not so thick that the flexible substrate is not severable.

The glass layer may particularly have a thickness and transparency selected as described in U.S. Patent Application Serial No. 09/493,958 titled "Multi-Featured Arrays With Reflective Coating" filed Jan. 28, 2000 by Andreas Dorsel, *et al*. while the reflective layer may meet the reflectivity requirements in relation to the illuminating light as mentioned in that application. For example, the reflective layer may reflect at least about 10% of the incident light, or at least about 20%, about 50%, about 80% or at least about 90%, or even at least about 95%, of the incident light. As mentioned previously, this and the other references cited herein are incorporated into this application by reference.

In the above configuration of the flexible substrate, the use of a glass layer allows the use of conventional chemistries, as discussed above, for substrate coating, feature fabrication, and array usage (for example, hybridization in the case of polynucleotide arrays). Such chemistries are well known for arrays on glass substrates, as described in the references cited herein and elsewhere. Furthermore, using a reflective layer not only can provide the useful characteristics mentioned in the above referenced patent application Serial No. 09/493,958, but can avoid undesirable optical characteristics of the plastic base layer (for example, undesirable fluorescence, and in some instances, excessive heating and possible melting of the substrate). This allows for the ability to use base layers of a material that may have a high fluorescence and/or high absorbance of incident light. For example, the plastic base layer may have a fluorescence of at least about five or about ten (or even at least: about twenty, about fifty, about one-hundred, or about two-hundred) reference units, and/or an absorbance of the illuminating light used to read arrays of at least about 5%, about 10%, about 20%, or about 50% (or even at least about 70%, about 90% or about 95%).

Use of a non-reflective opaque layer (for example, a suitably dyed plastic or other layer) in place of reflective layer also allows the use of the foregoing materials for a base layer although in such a case some heat may then be generated in the opaque layer. A reflective or non-reflective opaque layer at the position of the base layer may block at least about 10% of the illuminating light incident on a front surface for reading arrays, and even at least about 20%, about 50%, or about 80% (or at least about 90% or about 95%) of the illuminating light. A non-reflective opaque layer may reflect less than about 95%, about 90%, about 80%, or about 50% (or even less than about 10%) of the illuminating light. Where neither a reflective layer nor other opaque layer is present, it will be preferable to employ a base layer that emits low fluorescence upon illumination with the excitation light, at least in the situation where the array is read by detecting fluorescence. The base layer in this case may emit less than about two hundred, about one hundred, about fifty, or about twenty (or even less than about ten or about five) reference units. Additionally in this case, the base layer is preferably relatively transparent to reduce the absorption of the incident illuminating laser light and subsequent heating if the focused laser beam travels too slowly over a region. For example, the base layer may transmit at least about 5%, about 10%, about 20%, or about 50% (or even at least about 70%, about 90%, or about 95%), of the illuminating light incident on the front surface. Note that all reflection and absorbance measurements herein, unless the contrary is indicated, are made with reference to the illuminating light incident on a front surface for reading arrays and may be measured across the entire integrated spectrum of such illuminating light or alternatively at 532 nm or 633 nm.

The invention has particular application to linear arrays of oligomers or polymers. The oligomer or polymer is a chemical entity that contains a plurality of monomers. It is generally accepted that the term "oligomers" is used to refer to a species of polymers. The terms "oligomer" and "polymer" may be used interchangeably herein. Polymers usually comprise at least two monomers. Oligomers generally comprise about 6 to about 20,000 monomers, preferably, about 10 to about 10,000, more preferably about 15 to about 4,000 monomers. Examples of polymers include polydeoxyribonucleotides, polyribonucleotides, other polynucleotides that are C-glycosides of a purine or pyrimidine base, or other modified polynucleotides, polypeptides, polysaccharides, and other chemical entities that contain repeating units of like chemical structure. Exemplary of oligomers are oligonucleotides and peptides.

A monomer is a chemical entity that can be covalently linked to one or more other such entities to form an oligomer or polymer. Examples of monomers include nucleotides, amino acids, saccharides, peptoids, and the like and subunits comprising nucleotides, amino acids, saccharides, peptoids and the like. The subunits may comprise all of the same component such as, for example, all of the same nucleotide or amino acid, or the subunit may comprise different components such as, for example, different nucleotides or different amino acids. The subunits may comprise about 2 to about 2000, or about 5 to about 200, monomer units. In general, the monomers have first and second sites (e.g., C-termini and N-termini, or 5' and 3' sites) suitable for binding of other like monomers by means of standard chemical reactions (e.g., condensation, nucleophilic displacement of a leaving group, or the like), and a diverse element that distinguishes a particular monomer from a different monomer of the same type (e.g., an amino acid side chain, a nucleotide base, etc.). The initial substrate-bound, or support-bound, monomer is generally used as a building block in a multi-step synthesis procedure to form a complete ligand, such as in the synthesis of oligonucleotides, oligopeptides, oligosaccharides, etc. and the like.

A biomonomer references a single unit, which can be linked with the same or other biomonomers to form a biopolymer (for example, a single amino acid or nucleotide with two linking groups one or both of which may have removable protecting groups). A biomonomer fluid or biopolymer fluid reference a liquid containing either a biomonomer or biopolymer, respectively (typically in solution).

A biopolymer is a polymer of one or more types of repeating units. Biopolymers are typically found in biological systems and particularly include polysaccharides (such as carbohydrates), and peptides (which term is used to include polypeptides, and proteins whether or not attached to a polysaccharide) and polynucleotides as well as their analogs such as those compounds composed of or containing amino acid analogs or non-amino acid groups, or nucleotide analogs or non-nucleotide groups. This includes polynucleotides in which the conventional backbone has been replaced with a non-naturally occurring or synthetic backbone, and nucleic acids (or synthetic or naturally occurring analogs) in which one or more of the conventional bases has been replaced with a group (natural or synthetic) capable of participating in Watson-Crick type hydrogen bonding interactions.

Polynucleotides are compounds or compositions that are polymeric nucleotides or nucleic acid polymers. The polynucleotide may be a natural compound or a synthetic compound. Polynucleotides include oligonucleotides and are comprised of natural nucleotides such as ribonucleotides and deoxyribonucleotides and their derivatives although unnatural nucleotide mimetics such as 2'-modified nucleosides, peptide nucleic acids and oligomeric nucleoside phosphonates are also used. The polynucleotide can have from about 2 to 5,000,000 or more nucleotides. Usually, the oligonucleotides are at least about 2 nucleotides, usually, about 5 to about 100 nucleotides, more usually, about 10 to about 50 nucleotides, and may be about 15 to about 30 nucleotides, in length. Polynucleotides include single or multiple stranded configurations, where one or more of the strands may or may not be completely aligned with another.

A nucleotide refers to a sub-unit of a nucleic acid and has a phosphate group, a 5 carbon sugar and a nitrogen containing base, as well as functional analogs (whether synthetic or naturally occurring) of such sub-units which in the polymer form (as a polynucleotide) can hybridize with naturally occurring polynucleotides in a sequence specific manner analogous to that of two naturally occurring polynucleotides. For example, a "polynucleotide" includes DNA (including cDNA), RNA, oligonucleotides, and PNA and other polynucleotides as described in US 5,948,902 and references cited therein (all of which are incorporated herein by reference), regardless of the source. An "oligonucleotide" generally refers to a nucleotide multimer of about 10 to 100 nucleotides in length, while a "polynucleotide" includes a nucleotide multimer having any number of nucleotides.

The devices and methods of the present invention are particularly useful where the chemical compounds of the array are oligonucleotides and such oligonucleotide arrays are severed by the user and employed for determinations of polynucleotides.

As mentioned above, biopolymer arrays can be fabricated by depositing previously obtained biopolymers (such as from synthesis or natural sources) onto a substrate, or by *in situ* synthesis methods. The *in situ* synthesis methods include those described in U.S. Pat. No. 5,449,754 for synthesizing peptide arrays, as well as WO 98/41531 and the references cited therein for synthesizing polynucleotides (specifically, DNA). Such *in situ* synthesis methods can be basically regarded as repeating at each spot the sequence of: (a) deprotecting any previously deposited monomer so that it can now link with a subsequently deposited protected monomer; and (b) depositing a droplet of another protected monomer for linking. Different monomers may be deposited at different regions on the substrate during any one iteration so that the different regions of the completed array will have different desired biopolymer sequences. One or more intermediate further steps may be required in each iteration, such as oxidation, capping and washing steps. The deposition methods basically involve depositing biopolymers at predetermined locations on a substrate, which are suitably activated such that the biopolymers can link thereto. Biopolymers of different sequence may be deposited at different regions of the substrate to yield the completed array. Washing or other additional steps may also be used. Reagents used in typical in situ synthesis are water sensitive, and thus the presence of moisture should be eliminated or at least minimized.

The in situ method for fabricating a polynucleotide array typically follows, at each of the multiple different addresses at which features are to be formed, the same conventional iterative sequence used in forming polynucleotides from nucleoside reagents on a substrate by means of known chemistry. This iterative sequence is as follows: (a) coupling a selected nucleoside through a phosphite linkage to a functionalized substrate in the first iteration, or a nucleoside bound to the substrate (i.e. the nucleoside-modified substrate) in subsequent iterations; (b) optionally, but preferably, blocking unreacted hydroxyl groups on the substrate bound nucleoside; (c) oxidizing the phosphite linkage of step (a) to form a phosphate linkage; and (d) removing the protecting group ("deprotection") from the now substrate bound nucleoside coupled in step (a), to generate a reactive site for the next cycle of these steps. The functionalized substrate (in the first cycle) or deprotected coupled nucleoside (in subsequent cycles) provides a substrate bound moiety with a linking group for forming the phosphite linkage with a next nucleoside to be coupled in step (a). A number of reagents involved in the above synthetic steps such as, for example, phosphoramidite reagents, are sensitive to moisture and anhydrous conditions and solvents are employed. Final deprotection of nucleoside bases can be accomplished using alkaline conditions such as ammonium hydroxide, in a known manner.

The foregoing chemistry of the synthesis of polynucleotides is described in detail, for example, in Caruthers, Science 230: 281-285, 1985; Itakura, *et al*., Ann. Rev. Biochem. 53: 323-356; Hunkapillar, *et al*., Nature 310: 105-110, 1984; and in "Synthesis of Oligonucleotide Derivatives in Design and Targeted Reaction of Oligonucleotide Derivatives", CRC Press, Boca Raton, Fla., pages 100 *et seq*., U.S. Patent Nos. 4,458,066, 4,500,707, 5,153,319, and 5,869,643, EP 0294196, and elsewhere.

As mentioned above, various ways may be employed to produce an array of polynucleotides on the surface of a substrate. Such methods are known in the art. One *in situ* method employs pulse-jet technology to dispense the appropriate phosphoramidite reagents and other reagents onto individual sites on a surface of a substrate. Oligonucleotides are synthesized on a surface of a substrate *in situ* using phosphoramidite chemistry. Solutions containing nucleotide monomers and other reagents as necessary such as an activator, e.g., tetrazole, are applied to the surface of a substrate by means of thermal pulse-jet technology (although piezoelectric activated pulse jets might also be used, but in any event the pulse jets used must be constructed of materials chemically compatible with the solutions used). Individual droplets of reagents are applied to reactive areas on the surface using, for example, a thermal pulse-jet type nozzle. The surface of the substrate may have an alkyl bromide trichlorosilane coating to which is attached polyethylene glycol to provide terminal hydroxyl groups. These hydroxyl groups provide for linking to a terminal primary amine group on a monomeric reagent. Excess of non-reacted chemical on the surface is washed away in a subsequent step. For example, see U.S. Patent No. 5,700,637 and PCT WO 95/25116 and PCT application WO 89/10977.

Another approach for fabricating an array of biopolymers on a substrate using a biopolymer or biomonomer fluid and using a fluid dispensing head is described in U.S. Patent No. 6,242,266 (Schleifer, *et al*.). The head has at least one jet that can dispense droplets onto a surface of a substrate. The jet includes a chamber with an orifice and an ejector, which, when activated, causes a droplet to be ejected from the orifice. Multiple droplets of the biopolymer or biomonomer fluid are dispensed from the head orifice so as to form an array of droplets on the surface of the substrate.

In another embodiment (U.S. Patent No. 6,232,072) (Fisher) a method of, and apparatus for, fabricating a biopolymer array is disclosed. Droplets of fluid carrying the biopolymer or biomonomer are deposited onto a front side of a transparent substrate. Light is directed through the substrate from the front side, back through a substrate backside and a first set of deposited droplets on the first side to an image sensor.

An example of another method for chemical array fabrication is described in U.S. Patent No. 6,180,351 (Cattell). The method includes receiving from a remote station information on a layout of the array and an associated first identifier. A local identifier is generated corresponding to the first identifier and associated array. The local identifier is shorter in length than the corresponding first identifier. The addressable array is fabricated on the substrate in accordance with the received layout information.

As indicated above, in the present invention sections of the linear array are identified by some marking so that the user can sever the flexible substrate carrying the linear array into one or more segments or sections of choice for conducting a desired analysis. Such markings may be, for example, bar codes, labels, lines or symbols printed at the scribe lines, markings cut, molded or built into the housing at the time of manufacture and the like. The markings may be applied to the exterior of the housing of the flexible substrate by a suitable writing system, which is under the control of a processor. The writing system also includes a writer in the form of a printer that applies markings onto the exterior of the flexible substrate housing by printing them in the form of, for example, bar codes, directly onto the housing of the flexible substrate (or indirectly such as onto a label later attached to the substrate). Each marking is associated with a corresponding array segment or section. In this context "printing" is used to include any appropriate means of applying the markings, such as by ink, laser ablation, impressing, and the like.

The size of the markings is generally such that the markings are visible, which may include visible by the use of instruments such as, for example, microscopes, handheld magnifying lens, bar coder readers, and the like. For ease of use by the user, the markings should be visible to the naked eye. The size of the lines indicating the scribe points may be about 0.001 inches to about 0.01 inches, usually, about 0.002 inches to about 0.050 inches. Letter or number markings identifying the segment are generally readable at 4 to 6 point fonts, but preferably are made at least 8 point font.

The markings may include an identifier, which is generated and used as described in U.S. Patent No. 6,180,351 titled "Chemical Array Fabrication with Identifier". The identifiers may also optionally include a communication address that identifies the address of a remote location on communication channel from which one or more characteristics of an array will be communicated in response to a received communication of the associated identifier. Such remote location may be that of communication module or alternatively that of another accessible memory on a communication channel carrying the database of array characteristic data and associated identifiers. Examples of a communication address may be a telephone number, computer ID on a WAN, or an Internet Universal Resource Locator.

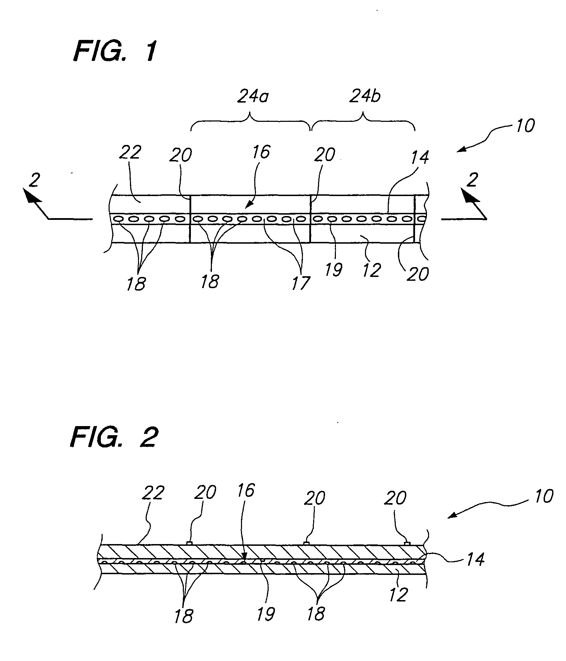

An example of a device in accordance with the present invention is depicted in Figs. 1 and 2. Device 10 comprises severable housing 12 having microchannel 14 contained therein. A linear array 16 of features 18 disposed in microchannel 14 on interior surface 19. Markings 20 are found on exterior surface 22 of housing 12. Markings 20 delineate segments 24 (24a, 24b and so forth) of linear array 16 comprising groups of one or more features of the linear array. Referring to Figs. 1-2, features 16 are separated by inter-feature regions 17. A typical linear array 16 may contain from about 100 to about 100,000 features. At least some, or all, of the features are of different compositions (for example, when any repeats of each feature composition are excluded the remaining features may account for at least about 5%, about 10%, or about 20% of the total number of features). Each feature carries a predetermined moiety (such as a particular polynucleotide sequence), or a predetermined mixture of moieties (such as a mixture of particular polynucleotides).

The devices in accordance with the present invention may be employed in various assays involving biopolymers. For example, following receipt by a user of a device 10 comprising a linear array 16 of the present invention, it will typically be severed into the segments or sections along markings 20 as desired by the user. The segment of choice is then exposed to a sample suspected of containing the analyte(s) of interest (for example, a fluorescent-labeled polynucleotide or protein-containing sample). The sample is usually introduced into the microchannel of the device through an opening that corresponds to the beginning and is moved along the channel by one of the approaches mentioned above. After the section of the linear array has been exposed to the sample and after a sufficient incubation period, the array is then read. Intervening washing steps may be employed to remove unbound materials prior to reading of the array.

The sample may be a trial sample, a reference sample, a combination of the foregoing, or a known mixture of components such as polynucleotides, proteins, polysaccharides and the like (in which case the arrays may be composed of features that are unknown such as polynucleotide sequences to be evaluated). The samples may be from biological assays such as in the identification of drug targets, single-nucleotide polymorphism mapping, monitoring samples from patients to track their response to treatment and/or assess the efficacy of new treatments, and so forth. For hybridization reactions, the sample generally comprises a target molecule that may or may not hybridize to a surface-bound molecular probe. The term "target molecule" refers to a known or unknown molecule in a sample, which will hybridize to a molecular probe on a substrate surface if the target molecule and the molecular probe contain complementary regions. In general, the target molecule is a "biopolymer," i.e., an oligomer or polymer. The present devices and methods have particular application to various processing steps involved with the aforementioned hybridization reactions.

An oligonucleotide probe may be, or may be capable of being, labeled with a reporter group, which generates a signal, or may be, or may be capable of becoming, bound to one a feature of the linear array. Detection of signal depends upon the nature of the label or reporter group. Commonly, binding of an oligonucleotide probe to a target polynucleotide sequence is detected by means of a label incorporated into the target. Alternatively, the target polynucleotide sequence may be unlabeled and a second oligonucleotide probe may be labeled. Binding can be detected by separating the bound second oligonucleotide probe or target polynucleotide from the free second oligonucleotide probe or target polynucleotide and detecting the label. In one approach, a sandwich is formed comprised of one oligonucleotide probe, which may be labeled, the target polynucleotide and an oligonucleotide probe that is or can become bound to a surface of a support. Alternatively, binding can be detected by a change in the signal-producing properties of the label upon binding, such as a change in the emission efficiency of a fluorescent or chemiluminescent label. This permits detection to be carried out without a separation step. Finally, binding can be detected by labeling the target polynucleotide, allowing the target polynucleotide to hybridize to a surface-bound oligonucleotide probe, washing away the unbound target polynucleotide and detecting the labeled target polynucleotide that remains. Direct detection of labeled target polynucleotide hybridized to surface-bound oligonucleotide probes is particularly advantageous in the use of ordered arrays.

In one approach, cell matter is lysed, to release its DNA as fragments, which are then separated out by electrophoresis or other means, and then tagged with a fluorescent or other label. The DNA mix is exposed to a segment of the linear array of oligonucleotide probes, whereupon selective attachment to matching probe sites takes place. The array is then washed and the result of exposure to the array is determined. In this particular example, the array is imaged by scanning the surface of the support so as to reveal for analysis and interpretation the sites where attachment occurred.

The signal referred to above may arise from any moiety that may be incorporated into a molecule such as an oligonucleotide probe for the purpose of detection. Often, a label is employed, which may be a member of a signal producing system. The label is capable of being detected directly or indirectly. In general, any reporter molecule that is detectable can be a label. Labels include, for example, (i) reporter molecules that can be detected directly by virtue of generating a signal, (ii) specific binding pair members that may be detected indirectly by subsequent binding to a cognate that contains a reporter molecule, (iii) mass tags detectable by mass spectrometry, (iv) oligonucleotide primers that can provide a template for amplification or ligation and (v) a specific polynucleotide sequence or recognition sequence that can act as a ligand such as for a repressor protein, wherein in the latter two instances the oligonucleotide primer or repressor protein will have, or be capable of having, a reporter molecule and so forth. The reporter molecule can be a catalyst, such as an enzyme, a polynucleotide coding for a catalyst, promoter, dye, fluorescent molecule, chemiluminescent molecule, coenzyme, enzyme substrate, radioactive group, a small organic molecule, amplifiable polynucleotide sequence, a particle such as latex or carbon particle, metal sol, crystallite, liposome, cell, etc., which may or may not be further labeled with a dye, catalyst or other detectable group, a mass tag that alters the weight of the molecule to which it is conjugated for mass spectrometry purposes, and the like.

The signal may be produced by a signal producing system, which is a system that generates a signal that relates to the presence or amount of a target polynucleotide in a medium. The signal producing system may have one or more components, at least one component being the label. The signal producing system includes all of the reagents required to produce a measurable signal. The signal producing system provides a signal detectable by external means, by use of electromagnetic radiation, desirably by visual examination. Signal-producing systems that may be employed in the present invention are those described more fully in U.S. Patent Nos. 6,558,908, 6,251,588, 6,235,483 and 6,132,997, the relevant disclosure of which is incorporated herein by reference.

The section of the linear array and the liquid sample are maintained in contact for a period of time sufficient for the desired chemical reaction to occur. The conditions for a reaction, such as, for example, period of time of contact, temperature, pH, salt concentration and so forth, are dependent on the nature of the chemical reaction, the nature of the chemical reactants including the liquid samples, and the like. The conditions for binding of members of specific binding pairs are generally well known and will not be discussed in detail here. The conditions for the various processing steps are also known in the art.

The linear arrays prepared as described above are particularly suitable for conducting hybridization reactions. Such reactions are carried out on a substrate or support comprising a plurality of features relating to the hybridization reactions. The substrate is exposed to liquid samples and to other reagents for carrying out the hybridization reactions. The support surface exposed to the sample is incubated under conditions suitable for hybridization reactions to occur.

After the appropriate period of time of contact between the liquid sample and the segment of the linear array, the contact is discontinued and various processing steps are performed. If desired, to increase the likelihood of specific complex formation, the sample and the probes can be passed through the device multiple times. Unbound detector probes and non-specifically bound sample components can then be washed from the device by, e.g., application of a wash fluid or the like, which is moved through the device by one of the aforementioned methods.

Detection of labels on the binding elements of the device corresponding to the particular specific binding pairs can be used as a measure of the presence of the analytes in the sample. For example, in one approach, following the processing step(s), the segment of the linear array is moved to an examining device where the linear array is interrogated. The examining device may be a scanning device involving an optical system.

Reading of the array may be accomplished by illuminating the array and reading the location and intensity of resulting fluorescence at each feature of the array. For example, a scanner may be used for this purpose where the scanner may be similar to, for example, the AGILENT MICROARRAY SCANNER available from Agilent Technologies Inc, Palo Alto, CA. Other suitable apparatus and methods are described in U.S. patent applications: Serial No. 09/846,125 "Reading Multi-Featured Arrays" by Dorsel, *et al*.; and U.S. Patent No. 6,406,849. The relevant portions of these references are incorporated herein by reference. However, arrays may be read by methods or apparatus other than the foregoing, with other reading methods including other optical techniques (for example, detecting chemiluminescent or electroluminescent labels) or electrical techniques (where each feature is provided with an electrode to detect hybridization at that feature in a manner disclosed in U.S. Patent Nos. 6,221,583 and 6,251,685, and elsewhere).

Results from the reading may be raw results (such as fluorescence intensity readings for each feature in one or more color channels) or may be processed results such as obtained by rejecting a reading for a feature that is below a predetermined threshold and/or forming conclusions based on the pattern read from the array (such as whether or not a particular target sequence may have been present in the sample). The results of the reading (processed or not) may be forwarded (such as by communication) to a remote location if desired, and received there for further use (such as further processing).

When one item is indicated as being "remote" from another, this means that the two items are at least in different buildings and may be at least one mile, ten miles, or at least one hundred miles apart. "Communicating" information references transmitting the data representing that information as electrical signals over a suitable communication channel (for example, a private or public network). "Forwarding" an item refers to any means of getting that item from one location to the next, whether by physically transporting that item or otherwise (where that is possible) and includes, at least in the case of data, physically transporting a medium carrying the data or communicating the data.

All publications and patent applications cited in this specification are herein incorporated by reference as if each individual publication or patent application were specifically and individually indicated to be incorporated by reference, except insofar as they may conflict with those of the present application (in which case the present application prevails). Methods recited herein may be carried out in any order of the recited events, which is logically possible, as well as the recited order of events.

The aforementioned description includes theories and mechanisms by which the invention is thought to work. It should be noted, however, that such proposed theories and mechanisms are not required and the scope of the present invention should not be limited by any particular theory and/or mechanism.

Although the foregoing invention has been described in some detail by way of illustration and example for purposes of clarity of understanding, it will be readily apparent to those of ordinary skill in the art in light of the teachings of this invention that certain changes and modifications may be made thereto without departing from the scope of the appended claims. Furthermore, the foregoing description, for purposes of explanation, used specific nomenclature to provide a thorough understanding of the invention. However, it will be apparent to one skilled in the art that the specific details are not required in order to practice the invention. Thus, the foregoing descriptions of specific embodiments of the present invention are presented for purposes of illustration and description; they are not intended to be exhaustive or to limit the invention to the precise forms disclosed. Many modifications and variations are possible in view of the above teachings. The embodiments were chosen and described in order to explain the principles of the invention and its practical applications and to thereby enable others skilled in the art to utilize the invention.

The disclosures in United States patent application no. 10/640,801, from which this application claims priority, and in the abstract accompanying this application are incorporated herein by reference.

## Claims

1. A device (10) comprising:
(a) a severable housing (12),
(b) a linear array (16) of features) comprising chemical compounds within said housing, and
(c) markings (20) on said housing indicating segments (24) of said linear array comprising groups of one or more features of said linear array.

2. A device according to Claim 1 wherein said housing is a channel (14) in a microfluidic system.

3. A device according to Claim 1 or 2 wherein said features are biopolymers.

4. A device according to any preceding Claim wherein said housing is formed from a flexible material.

5. A device according to any preceding Claim wherein said linear array comprises at least ten features.

6. A method for conducting an assay for one or more analytes suspected of being in a sample, said method comprising:
(a) severing said device of any of Claims 1 to 5 to obtain a segment comprising features for specifically identifying said one or more analytes,
(b) contacting said features with said sample and
(c) determining which of said analytes have become bound to said features.

7. A method according to Claim 6 wherein said features are binding partners for each of said analytes or for a complex of an analyte with a respective binding partner for said analyte.

8. A method for conducting an assay for one or more biopolymers suspected of being in a sample, said method comprising:
(a) severing a device(10) to obtain a segment (24) comprising features (18) for specifically identifying said one or more biopolymers, wherein said device comprises:
(i) a severable housing (12),
(ii) a linear array (16) of said features (18) within said housing, and
(iii) markings (20) on said housing indicating segments (24) comprising said segment,
(b) contacting said features of said segment with said sample and
(c) determining which of said biopolymers have become bound to said features.

9. A method according to Claim 8 wherein said features are binding partners for each of said biopolymers.

10. A method of preparing a device (10) for conducting an assay for one or more analytes, said method comprising:
(a) forming a linear microarray (16) of features (18) within a severable housing (12) wherein at least a portion of said features is for detecting said one or more analytes and
(b) marking said housing to indicate segments (24), at least one of which segments comprises said portion of features.

11. A method of preparing a device (10) for conducting an assay for one or more analytes, said method comprising:
(a) forming a linear array (16) of features (18) on a flexible substrate wherein at least a portion of said features is for detecting said one or more analytes, and
(b) sealing said flexible substrate to form a channel (14) comprising said linear array.

12. A method according to Claim 11 wherein said sealing is carried out by folding said flexible substrate to form said channel or by bonding a second severable material to said flexible substrate to form said channel.
